# FASCICULE DE BREVET EUROPEEN

(11) **EP 0 751 764 B1**
(45) Date de publication et mention de la délivrance du brevet: **12.12.2001**
(21) Numéro de dépôt: 95914397.5
(22) Date de dépôt: 22.03.1995
(51) Int. Cl.: A61K 31/07, A61K 9/107, A61K 7/48

(54) **COMPOSITION CONTENANT DU RETINAL**
RETINAL ENTHALTENDES ARZNEIMITTEL
RETINAL-CONTAINING COMPOSITION

(30) Priorité: 22.03.1994 FR 9403339
(43) Date de publication de la demande: 08.01.1997
(73) Titulaire: Pierre Fabre Dermo-Cosmetique, 92100 Boulogne (FR)
(72) Inventeur: COUVAL, Emmanuelle, F-69300 Caluire et Cuire (FR); PEYROT, Nicole, F-31400 Toulouse (FR); FIRMINO, Nathalie, F-31520 Ramonville-Saint-Agne (FR); JAMMES, Henri, F-31400 Toulouse (FR); CLAIRAND, Valérie, F-31500 Toulouse (FR)
(74) Mandataire: Ahner, Francis
(86) Numéro de dépôt international: FR9500350
(87) Numéro de publication internationale: WO9525507

(56) Documents cités:
- EP-A- 0 094 771
- EP-A- 0 330 496
- EP-A- 0 408 370
- WO-A-93/00085
- FR-A- 2 247 203

## Description

La présente invention se rapporte à de nouvelles formes galéniques adaptées à une application topique, et contenant du rétinal.

Les produits de soins contenant des rétinoïdes sont devenus un centre d'intérêt ces dernières années. L'acide rétinoïque, aussi connu sous le nom de vitamine A acide ou trétinoïne, est utilisé dans le traitement de l'acné et les compositions contenant de la vitamine A acide, sont nombreux et variés.

Plus récemment, d'autres applications des rétinoïdes ont été mises en avant, tel que le vieillissement actinique. En effet, les personnes qui se sont beaucoup exposées au soleil pendant leur enfance présentent, à l'âge adulte, les caractéristiques cutanées suivantes : peau ridée, burinée, jaune, relachée, rugueuse, sèche, tachetée (hyperpigmentation) et diverses grosseurs prémalignes. Ces phénomènes sont plus marqués chez les personnes à peau claire, qui brûlent souvent au soleil et bronzent peu.

Avant que n'apparaissent cliniquement ces manifestations cutanées, on peut constater microscopiquement de sérieuses altérations de l'épiderme et du derme chez le sujet jeune qui s'est beaucoup exposé.

Le brevet U.S. -A- 4 603 146 décrit le traitement de la peau abîmée par l'exposition solaire, à l'aide d'une préparation contenant de la vitamine A acide dans un excipient émollient. Plus tard, dans le brevet U.S. -A- 4 877 805 il est enseigné que les rétinoïdes peuvent être utilisés pour prévenir et restaurer les dommages provoqués par le soleil sur la peau humaine.

On sait également que l'utilisation du rétinal (vitamine A aldéhyde) est préférée à celle de l'acide rétinoïque en raison de sa meilleure tolérance cutanée. En effet, le rétinal (vitamine A aldéhyde) apparaît naturellement dans le métabolisme humain : il est notamment utilisé dans des traitements destinés à améliorer la vision.

Toutefois, il s'agit d'un composé présentant une mauvaise stabilité physico-chimique ; sa formulation sous une forme présentant de bonnes qualités organoleptiques ainsi que de bonnes caractéristiques de conservation n'a pas été résolue de façon satisfaisante à ce jour.

Dans le cadre de la présente invention, le rétinal actif peut se présenter sous forme 13-cis, 13-trans, ou tous autres mélanges de ces isomères.

Lors du stockage, d'autres formes ont tendance à apparaître, comme le 9-cis-rétinal, le 11-cis-rétinal, ou des produits de condensations de type polymère, qui sont inactives.

Le brevet US 4 826 828 a proposé l'utilisation de silicones volatils et d'éthanol pour la préparation de compositions contenant du rétinol ; ces préparations peuvent être diluées avant application par formation d'une émulsion eau/huile.

Le brevet US 4 720 353 décrit des émulsions eau/huile stabilisées par un organopolysiloxane particulier.

Toutefois, ces formulations ne donnent pas des résultats satisfaisants à la conservation.

La demande WO 93/00085 décrit des formulations de rétinoïdes sous forme d'émulsion eau/huile, stabilisées par un système complexe comprenant un agent chélatant et des anti-oxydants hydrosolubles et liposolubles.

Il s'agit de formulations comportant de nombreux paramètres difficiles à mettre en oeuvre. En outre les émulsions eau/huile sont mal adaptées à une application topique, particulièrement en cosmétologie.

C'est pourquoi la présente invention a pour objet une composition dermocosmétologique contenant du rétinal, caractérisée en ce qu'elle est constituée d'une émulsion huile dans eau stabilisée essentiellement par un ou plusieurs anti-oxydants liposolubles, le rapport des concentrations anti-oxydant liposoluble/rétinal étant compris entre 0,1 et 0,6.

Des émulsions huile/eau selon la présente invention possèdent de bonnes capacités d'étalement, sans toucher gras. Le système de stabilisation spécifique permet de limiter les phénomènes de dégradation dans le temps observés avec les émulsions huile/eau classiques.

Les anti-oxydants particulièrement adaptés à l'obtention d'un système de stabilisation selon l'invention peuvent être choisis dans le groupe comprenant : le Butyl Hydroxy Toluène (BHT), le Butyl Hydroxy Anisole (BHA), le Palmitate d'Ascorbyle, l'Alpha-Tocophérol et ses esters, l'Acide Citrique, le Gallate de Propyle, et/ou leurs mélanges.

L'Alpha-Tocophérol peut notamment être sous forme d'acétate.

Le rétinal présent dans la composition est incorporé dans la phase huileuse (interne) de l'émulsion. Le rétinal sera ainsi mieux protégé de l'oxygène de l'air que s'il se trouvait en phase externe (dans une émulsion eau/huile par exemple) et donc directement en contact avec ce dernier.

En général, la concentration en rétinal dans la composition selon l'invention est d'au moins 0,01% en poids, et peut atteindre jusqu'à environ 1% en poids de la composition totale.

Les concentrations préférées pour le rétinal sont comprises entre 0,04% et 0,06% en poids, et sera plus particulièrement d'environ 0,05%.

De préférence, la concentration en anti-oxydants liposolubles dans la phase huileuse est comprise entre 0,005 % et 0,5 % en poids.

Ces concentrations varieront en fonction de l'anti-oxydant utilisé. Par exemple, le BHT ou le BHA seront présents chacun à environ 0,01% à 0,03% en poids. Les concentrations d'Alpha-Tocophérol ou de ses esters peuvent atteindre 0,5%, et sont de préférence supérieures à 0,05% en poids.

Un facteur important pour la stabilité des émulsions est le rapport des concentrations en anti-oxydants liposolubles aux concentrations en rétinal. Des résultats particulièrement avantageux en terme de stabilité au stockage sont obtenus lorsque le rapport des concentrations anti-oxydant liposoluble / rétinal est compris entre 0,1 et 0,6, notamment quand le rapport est d'environ 0,4.

La phase huileuse peut contenir des huiles minérales et/ou organiques, polaires ou non, cires, esters et silicones. Selon les caractéristiques physiques souhaitées, les compositions selon l'invention peuvent également contenir des agents tensio-actifs, des épaississants gélifiants, parfums et colorants.

Le pH des compositions est de préférence légèrement acide ou proche de la neutralité, en vue de son application sur la peau ; des compositions conformes à l'invention ont notamment un pH compris entre 6 et 7,5.

Selon l'un de ses aspects l'invention a pour objet une composition huile/eau, caractérisée en ce qu'elle contient du rétinal à une concentration comprise entre 0,04 % et 0,06 % en poids, de préférence d'environ 0,05 % et du BHT à une concentration comprise entre 0,01 % et 0,03 %, de préférence d'environ 0,02 % en poids.

Selon encore un autre aspect, l'invention concerne une composition huile/eau, caractérisée en ce qu'elle contient du rétinal à une concentration comprise entre 0,04 % et 0,06 % en poids, de préférence d'environ 0,05 % et du Gallate de Propyle à une concentration comprise entre 0,01 % et 0,03 %, de préférence d'environ 0,02 % en poids.

L'invention comprend l'utilisation cosmétique des émulsions huile/eau ainsi définies.

Elle comprend également l'utilisation à titre de médicament des compositions à base du rétinal présentant une ou plusieurs des caractéristiques ci-dessus.

Enfin, la présente invention s'étend également à un procédé de préparation des compositions huile/eau, qui comporte les étapes suivantes:
a) on met le rétinal en solution dans une ou plusieurs huiles organiques et/ou minérales,
b) on ajoute un ou plusieurs anti-oxydants liposolubles à la solution,
c) on émulsionne la solution obtenue dans une solution aqueuse et/ou hydro-alcoolique, pour obtenir une émulsion huile/eau.

Les exemples qui suivent sont destinés à illustrer l'invention sans aucunement en limiter la portée.

### Exemple 1 : Etude qualitative de stabilisation du rétinal

Dans un premier temps, différents anti-oxydants lipophiles ont été testés afin de définir celui qui stabilise le mieux le rétinal :

Les anti-oxydants testés sont :

| | | |
|---|---|---|
| . BHA | Butyl Hydroxy Anisole | |
| | | |
| . BHT | Butyl Hydroxy Toluène | |
| | | |
| . GALLATE de PROPYLE | | |
| | | |
| . RONOXAN ^{(R)} A | Alpha tocophérol | 5% |
| | Palmitate d'ascorbyle | 25% |
| | Q.S.P. lécithine | |
| | | |
| . RONOXAN ^{(R)} 582 | Palmitate d'ascorbyle | 15% |
| | Mono et diglycéride | 85% |
| | | |
| . RONOXAN ^{(R)} 583 | Palmitate d'ascorbyle | 11% |
| | Alpha tocophérol | 11% |
| | Acide citrique | 3% |
| | Mono et diglycéride | 75% |
| . ACETATE d'ALPHA TOCOPHEROL | | |
| | | |
| . TOCOPHEROL | | |

Le Rétinal (vitamine A aldéhyde) est solubilisé à 0,05 % dans 2 huiles : l'une polaire : Miglyol ^{(R)} 812 (triglycérides caprique/caprylique), l'autre non polaire : Cosbiol ^{(R)} (Squalane).

### Résultats

**TABLEAU 1**

| Solution de Rétinal (vitamine A aldéhyde) à 0,05 % dans une huile polaire (Miglyol ^{(R)} 812 : triglycérides caprique/caprylique). | | |
|---|---|---|
| Anti-oxydant | Stockage à T° ambiante ≃ 20° C | Quantité de Trans Rétinal restant (%) |
| BHA 0,01 % | T 1 mois | 92,8 % |
| | T 6 mois | 76% |
| BHT 0,01 % | T 1 mois | 92,4 % |
| | T 6 mois | 78,4 % |
| GALLATE de PROPYLE 0,01 % | T 1 mois | 91,4 % |
| | T 6 mois | 73,7 % |
| RONOXAN ^{(R)} A 0,1 % | T 1 mois | 90 % |
| | T 6 mois | 60,2 % |
| RONOXAN ^{(R)} 582 0,1% | T1 mois | 94,1 % |
| | T 6 mois | 78,4% |
| RONOXAN ^{(R)} 583 0,1% | T1 mois | 88,2 % |
| | T 6 mois | 75,2% |
| ACETATE D'ALPHA TOCOPHEROL + TOCOPEROL 0,2 % | T 1 mois | 90,7 % |
| | T 6 mois | 73,8 % |
| Témoin sans anti-oxydant | T1 mois | 81,3% |
| | T6 mois | 41,4% |

**TABLEAU 2**

| Solution de Rétinal (vitamine A aldéhyde) à 0,05 % dans une huile non polaire (Cosbiol : Squalane) | | |
|---|---|---|
| Anti-oxydant | Stockage à T° ambiante ≃ 20°C | Quantité de Trans Rétinal restant (%) |
| BHA 0,01 % | T 1 mois | 98,4 % |
| | T 6 mois | 75 % |
| BHT 0,01 % | T 1 mois | 94,8 % |
| | T 6 mois | 73,5% |
| GALLATE de PROPYLE 0,01 % | T 1 mois | 97,3 % |
| | T 6 mois | 76,4 % |
| RONOXAN ^{(R)} A 0,1 % | T 1 mois | 90,55 % |
| | T 6 mois | 69,6 % |
| RONOXAN ^{(R)} 582 0,1 % | T 1 mois | 91,44 % |
| | T 6 mois | 69,7 % |
| RONOXAN ^{(R)} 583 0,1 % | T 1 mois | 98,2 % |
| | T 6 mois | 78,5 % |
| ACETATE D'ALPHA TOCOPHEROL+ TOCOPHEROL 0,2 % | T 1 mois | 91,9 % |
| | T 6 mois | 76,2 % |
| Témoin sans anti-oxydant | T1 mois | 85,6% |
| | T6 mois | 43,7% |

### Conclusion

L' étude qualitative des anti-oxydants lipophiles vis-à-vis de la stabilisation du Rétinal (vitamine A aldéhyde) met en évidence de bons résultats avec le RONOXAN 582, le RONOXAN 583, le GALLATE de PROPYLE, le BHA et le BHT, et ce, dans les 2 solvants Miglyol 812 ^{(R)} et Cosbiol.

### Exemple 2 : Etude qualitative de protection d'un mélange de corps gras

Une étude systématique de protection vis-à-vis de l'oxydation d'un mélange de corps gras et de son système émulsionnant, a été réalisée avec les mêmes anti-oxydants que ceux décrits dans le paragraphe précédent.

La phase grasse retenue correspond à un mélange cosmétique optimal, qui sera introduit ultérieurement au sein d'une phase aqueuse pour réaliser une émulsion huile dans eau.

La composition de cette phase est la suivante :

| | |
|---|---|
| . Neobee 18 ^{(R)} huile de Carthame hybride | 10 g |
| . Huile de vaseline épaisse | 5 g |
| . Cremophor RH 40 ^{(R)} | |
| (huile de Ricin hydrogénée et éthoxylée) | 1 g |
| . Simulsol 165 ^{(R)} | |
| (monostéarate de glycérol POE) | 8,5 g |
| . Cire E (microcristalline) | 1,5 g |
| . Acetulan ^{(R)} (alcools de lanoline acétylés) | 2 g |
| . Neobee M5 ^{(R)} (triglycérides de coco) | 9 g |
| . Solulan PB 10 ^{(R)} (éther de lanoline propoxylé) | 1,5 g |
| . 2 phénoxyéthanol | 0,5 g |
| . Parahydroxybenzoate de méthyle | 0,2 g |
| . Parahydroxybenzoate de butyle | 0,10 g |
| | |
| Total | 39,3 g |

N.B. La phase aqueuse qui sera rajoutée ultérieurement constituera 60,7 % de la formule, faisant au total 100 %.

### Résultats

l'indice de péroxyde a été dosé au temps 0 et au bout de 6 mois de stockage à température ambiante voisine de 20° C.

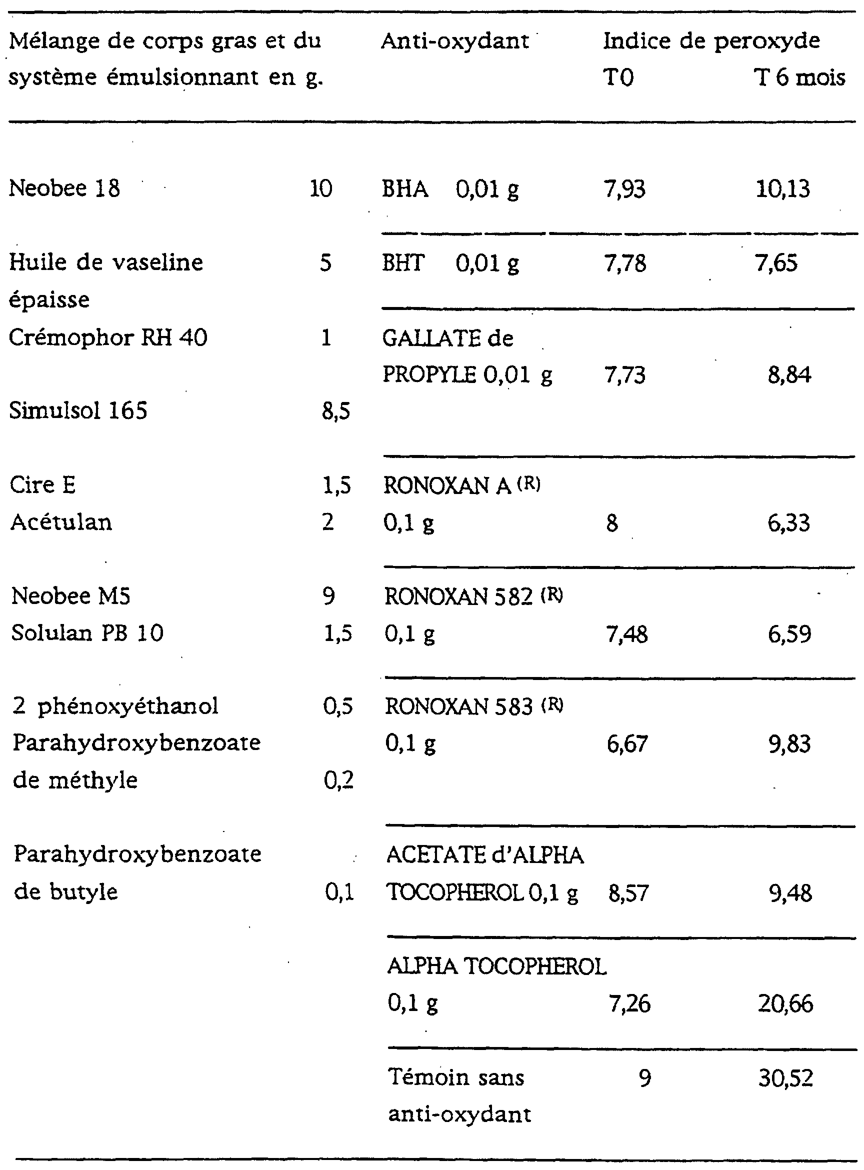

### Conclusion

Cette étude de protection vis-à-vis de l'oxydation, d'un mélange de corps gras, met en évidence de bons résultats avec le RONOXAN (R) 582, le RONOXAN (R) 583, le Gallate de Propyle, le BHA et l'Acétate d' Alpha Tocophérol et, notamment, le BHT.

### Exemple 3 : Etude quantitative avec le BHT

Une étude quantitative de stabilisation de rétinal dans une émulsion huile dans eau, a été menée avec le BHT (Butyl Hydroxy Toluène) qui avait donné de très bons résultats dans l'étude précédente.

L'émulsion retenue a été conservée 6 mois à température ambiante (voisine de 20° C).

Le rétinal a été dosé dans l'émulsion à différents temps de conservation.

| Forme de l'émulsion huile dans eau | |
|---|---|
| ( Formule 1 Rétinal | 0,05 % |
| ( Formule 2 Rétinal | 0,025 % |

| Phase grasse | |
|---|---|
| BHT | 0,01 % |
| Neobee 18 | 10 % |
| Huile de vaseline épaisse | 5 % |
| Cremophor RH 40 | 1 % |
| Simulsol 165 | 8,50 % |
| Cire E | 1,50 % |
| Acétulan | 2 % |
| Neobee M 5 | 10 % |
| Solulan PB 10 | 1,50 % |
| 2 phénoxyéthanol | 0,50 % |
| Parahydroxybenzoate de méthyle | 0,20 % |
| Triéthanolamine | 0,30 % |

| Phase aqueuse | |
|---|---|
| Propylène glycol | 3 % |
| Carbopol 934 | 0,25 % |
| Eau purifiée qsp | 100 % |
| | pH: voisin de 7 |

| | Formule 1 | Formule 2 |
|---|---|---|
| | % du Trans rétinal restant | |
| T 1 mois | 98,9 % | 98,8 % |
| T 2 mois | 98 % | 96,9 % |
| T 3 mois | 96 % | 94,9 % |
| T 6 mois | 83 % | 92,9 % |

### Conclusion

Les excellents résultats enregistrés à 6 mois notamment avec la formule 2 mettent en évidence une bonne protection du rétinal par le BHT dans le rapport
0,01 / 0,025
BHT / rétinoïde

L'ordre de grandeur de ce rapport est conservé dans le produit fini.

Le rétinal a été déterminé par chromatographie liquide haute performance dans les conditions suivantes :

Appareil équipé d'une colonne de silice normale (5 µ), longueur 25 cm de diamètre interne 0,4 cm, d'un détecteur à longueur d'onde variable fixé à 365 nm.

La phase éluante est composée d'hexane à 99%, dioxanne, isopropanol dans les proportions 990-10-1. Son débit est de 1,5 ml/min.

L'échantillon à analyser est dissous dans le dioxanne, puis dans l'isopropanol et dans l'hexane dans les proportions de la phase éluante et à une concentration de 1 µg/ml. 20 µl de solution à analyser sont injectés dans le chromatographe en mode isocratique et à température ambiante, le rétinal sort avec un temps de rétention voisin de 7,5 min environ.

Les résultats HPLC sont reproductibles avec un coefficient de variation de 2,4%.

### Exemple 4 : Etude de la stabilisation du rétinal dans une émulsion huile dans eau

### Formule détaillée

| Phase aqaueuse | |
|---|---|
| Eau purifiée qsp | 100 % |
| Carbopol 934 | 0,25 % |
| Propylène glycol | 3 % |

| Phase grasse | |
|---|---|
| Neobee 18 | 10 % |
| Paraffine liquide | 5 % |
| Cremophor RH 40 | 1 % |
| Simulsol 165 | 8,50 % |
| Cire E paillettes | 1,50 % |
| Acétulan | 2 % |
| Neobee M 5 | 10 % |
| Solulan PB 10 | 1,50 % |
| BHT | 0,02 % |
| 2 phénoxyéthanol | 0,50 % |
| Parahydroxybenzoate de méthyle | 0,20 % |
| Triéthanolamine | 0,30 % |
| | |
| | pH : voisin de 7 |

### Résultats

L'émulsion a été conservée 18 mois à température ambiante (voisine de 20° C) et 6 mois à 40° C en vieillissement accéléré.

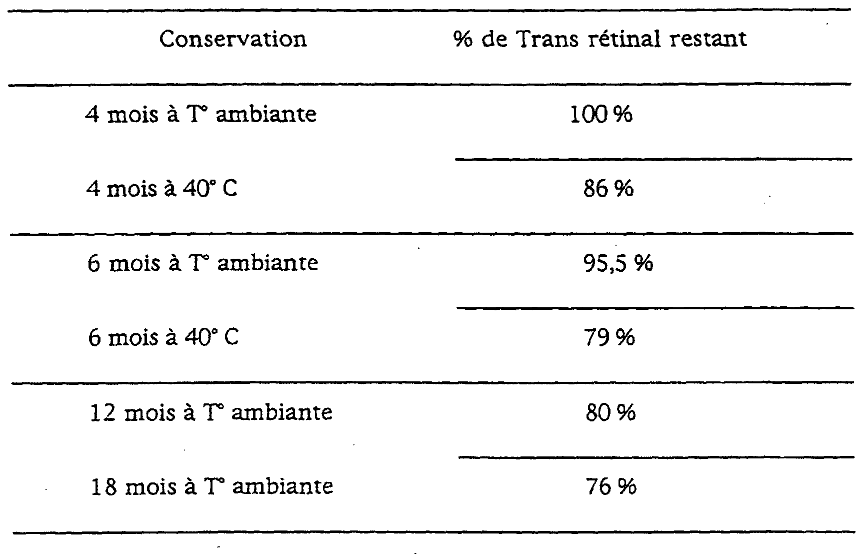

### Conclusion

L'émulsion proposée présente une excellente stabilité du Rétinal avec 95,5 % de Trans rétinal au bout de 6 mois à 40° C et 76 % au bout de 18 mois à température ambiante.

### Exemple 5

### Exemples de formulations

| **Formule A** | | |
|---|---|---|
| 1. | Rétinal (forme Trans) | 0,05 % |
| 2. | Huile de Carthame hybride (Neobee 18) | 10 % |
| 3. | Huile de Vaseline épaisse | 5 % |
| 4. | Triglycérides de Coco (Neobee M 5) | 10 % |
| 5. | Huile de Ricin hydrogénée éthoxylée (Cremophor RH 40) | 1 % |
| 6. | Monostéarate de glycérol/POE (Simulsol 165) | 8,5 % |
| 7. | Cire microcristalline (Cire E) | 1,5 % |
| 8. | Alcools de lanoline acétylés (Acétulan) | 2 % |
| 9. | Ether de lanoline propoxylé (Solulan PB 10) | 1,5 % |
| 10. | Butyl Hydroxy Toluène | 0,02 % |
| 11. | 2-Phénoxyéthanol | 0,5 % |
| 12. | Parahydroxybenzoate de propyle | 0,4 % |
| 13. | Parahydroxybenzoate de butyle | 0,2 % |
| 14. | Carbopol 934 | 0,25 % |
| 15. | Propylène glycol | 3 % |
| 16. | Triéthanolamine qsp pH≃6,5 | 0,3 % |
| 17. | Eau purifiée qsp | 100 % |
| | | |
| | | pH : voisin de 6,5 |

| **Formule B** | | |
|---|---|---|
| 1. | Rétinal (forme Trans) | 0,05 % |
| 2. | Huile de Carthame hybride (Neobee 18) | 10 % |
| 3. | Huile de Vaseline épaisse | 5 % |
| 4. | Triglycérides de Coco (Neobee M 5) | 10 % |
| 5. | Huile de Ricin hydrogénée éthoxylée (Cremophor RH 40) | 1 % |
| 6. | Monostéarate de glycérol/POE (Simulsol 165) | 8,5 % |
| 7. | Cire microcristalline (Cire E) | 1,5 % |
| 8. | Alcools de lanoline acétylés (Acétulan) | 2 % |
| 9. | Ether de lanoline propoxylé (Solulan PB 10) | 1,5 % |
| 10. | Butyl Hydroxy Anisole | 0,02 % |
| 11. | 2-Phénoxyéthanol | 0,5 % |
| 12. | Parahydroxybenzoate de propyle | 0,4 % |
| 13. | Parahydroxybenzoate de butyle | 0,2 % |
| 14. | Carbopol 934 | 0,25 % |
| 15. | Propylène glycol | 3 % |
| 16. | Triéthanolamine qsp pH≃6,5 | 0,3 % |
| 17. | Eau purifiée qsp | 100 % |
| | | |
| | | pH : voisin de 6,5 |

| **Formule C** | | |
|---|---|---|
| 1. | Rétinal (forme Trans) | 0,05 % |
| 2. | Huile de Carthame hybride (Neobee 18) | 10 % |
| 3. | Huile de Vaseline épaisse | 5 % |
| 4. | Triglycérides de Coco (Neobee M 5) | 10 % |
| 5. | Huile de Ricin hydrogénée éthoxylée (Cremophor RH 40) | 1 % |
| 6. | Monostéarate de glycérol/POE (Simulsol 165) | 8,5 % |
| 7. | Cire microcristalline (Cire E) | 1,5 % |
| 8. | Alcools de lanoline acétylés (Acétulan) | 2 % |
| 9. | Ether de lanoline propoxylé (Solulan PB 10) | 1,5 % |
| 10. | Ronoxan 582 (Palmitate d'ascorbyle) | 0,2 % |
| 11. | 2-Phénoxyéthanol | 0,5 % |
| 12. | Parahydroxybenzoate de propyle | 0,4 % |
| 13. | Parahydroxybenzoate de butyle | 0,2 % |
| 14. | Carbopol 934 | 0,25 % |
| 15. | Propylène glycol | 3 % |
| 16. | Triéthanolamine qsp pH≃6,5 | 0,3 % |
| 17. | Eau purifiée qsp | 100 % |
| | | |
| | | pH : voisin de 6,5 |

| **Formule D** | | |
|---|---|---|
| 1. | Rétinal (forme Trans) | 0,05 % |
| 2. | Huile de Carthame hybride (Neobee 18) | 10 % |
| 3. | Huile de Vaseline épaisse | 5 % |
| 4. | Triglycérides de Coco (Neobee M 5) | 10 % |
| 5. | Huile de Ricin hydrogénée éthoxylée (Cremophor RH 40) | 1 % |
| 6. | Monostéarate de glycérol/POE (Simulsol 165) | 8,5 % |
| 7. | Cire microcristalline (Cire E) | 1,5 % |
| 8. | Alcools de lanoline acétylés (Acétulan) | 2 % |
| 9. | Ether de lanoline propoxylé (Solulan PB 10) | 1,5 % |
| 10. | Gallate de propyle | 0,02 % |
| 11. | 2-Phénoxyéthanol | 0,5 % |
| 12. | Parahydroxybenzoate de propyle | 0,4 % |
| 13. | Parahydroxybenzoate de butyle | 0,2 % |
| 14. | Carbopol 934 | 0,25 % |
| 15. | Propylène glycol | 3 % |
| 16. | Triéthanolamine qsp pH≃6,5 | 0,3 % |
| 17. | Eau purifiée qsp | 100 % |
| | | pH: voisin de 6,5 pH : voisin de 6,5 |

## Revendications

1. Composition dermocosmétologique contenant du rétinal, sous forme 13-cis, 13-trans ou de tous autres mélanges de ces isomètres **caractérisée en ce qu'**elle est constituée d'une émulsion huile dans eau stabilisée essentiellement par un ou plusieurs anri-oxydants liposolubles, le rapport des concentrations anti-oxydant liposoluble / rétinal étant compris entre 0,1 et 0,6.

2. Composition dermocosmétologique selon la revendication 1, **caractérisée en ce que** l'anti-oxydant liposoluble est choisi dans le groupe comprenant : le Butyl Hydroxy Toluène (BHT), le Butyl Hydroxy Anisole (BHA), le Palmitate d'ascorbyle, l'Alpha tocophérol et ses esters, l'Acide citrique, le Gallate de Propyle, et/ou leurs mélanges.

3. Composition selon l'une des revendications 1 ou 2 **caractérisée en ce que** le rétinal est présent dans la phase huileuse.

4. Composition selon l'une des revendications 1 à 3 **caractérisée en ce que** la concentration en anti-oxydants liposolubles dans la phase huileuse est comprise entre 0,005 % et 0,5 % en poids.

5. Composition selon l'une des revendications 1 à 4 **caractérisée en ce que** la concentration en rétinal dans la composition est comprise entre 0,01 % et 1 % en poids.

6. Composition selon l'une des revendications 1 à 5, **caractérisée en ce que** le rapport des concentrations anti-oxydant liposoluble / rétinal est environ de 0,4.

7. Composition selon l'une des revendications 1 à 6 **caractérisée en ce qu'**elle contient du rétinal à une concentration comprise entre 0,04 % et 0.06 % en poids, de préférence d'environ 0,05 % et du BHT à une concentration comprise entre 0,01 % et 0,03 %, de préférence d'environ 0,02 % en poids.

8. Composition selon l'une des revendications 1 à 6 **caractérisée en ce qu'**elle contient du rétinal à une concentration comprise entre 0,04 % et 0,06 % en poids, de préférence d'environ 0,05 % et du Gallate de Propyle à une concentration comprise entre 0,01 % et 0,03 %, de préférence d'environ 0,02 % en poids.

9. Composition selon l'une des revendications 1 à 8, **caractérisée en ce que** le pH de la composition est compris entre 6 et 7,5.

10. Procédé de préparation d'une composition selon l'une des revendications 1 à 9, **caractérisé en ce qu'**il comprend les étapes suivantes :
a) on met le rétinal en solution dans une ou plusieurs huiles organiques et/ou minérales, cires, esters et silicones,
b) on ajoute un ou plusieurs anti-oxydants liposolubles à la solution,
c) on émulsionne la solution obtenue dans une solution aqueuse et/ou hydro-alcoolique, pour obtenir une émulsion huile/eau.

## Patentansprüche

1. Dermokosmetische Zusammensetzung, enthaltend Retinal in 13-cis-, 13-trans-Form oder in Form aller anderen Mischungen dieser Isomere, **dadurch gekennzeichnet, dass** sie aus einer Öl-in-Wasser-Emulsion zusammengesetzt ist, die im wesentlichen durch ein oder mehrere fettlösliche Antioxidantien stabilisiert ist, wobei das Verhältnis der Konzentrationen von fettlöslichem Antioxidans/Retinal zwischen 0,1 und 0,6 einschießlich liegt.

2. Dermokosmetische Zusammensetzung nach Anspruch 1, **dadurch gekennzeichnet, dass** das fettlösliche Antioxidans ausgewählt ist aus der Gruppe umfassend: Butylhydroxytoluol (BHT), Butylhydroxyanisol (BHA), Ascorbylpalmitat, alpha-Tocopherol und seine Ester, Citronensäure, Propylgallat und/oder deren Mischungen.

3. Zusammensetzung nach einem der Ansprüche 1 oder 2, **dadurch gekennzeichnet, dass** das Retinal in der Ölphase vorliegt.

4. Zusammensetzung nach einem der Ansprüche 1 bis 3, **dadurch gekennzeichnet, dass** die Konzentration an fettlöslichen Antioxidantien in der Ölphase zwischen 0,005 und 0,5 Gewichts-% einschließlich liegt.

5. Zusammensetzung nach einem der Ansprüche 1 bis 4, **dadurch gekennzeichnet, dass** die Konzentration an Retinal in der Zusammensetzung zwischen 0,01 und 1 Gewichts-% einschließlich liegt.

6. Zusammensetzung nach einem der Ansprüche 1 bis 5, **dadurch gekennzeichnet, dass** das Verhältnis der Konzentrationen fettlösliches Antioxidans/ Retinal etwa 0,4 beträgt.

7. Zusammensetzung nach einem der Ansprüche 1 bis 6, **dadurch gekennzeichnet, dass** sie Retinal in einer Konzentration von 0,04 bis 0,06 Gewichts-%, vorzugsweise etwa 0,05 Gewichts-%, und BHT in einer Konzentration von 0,01 bis 0,03, vorzugsweise etwa 0,02 Gewichts-% enthält.

8. Zusammensetzung nach einem der Ansprüche 1 bis 6, **dadurch gekennzeichnet, dass** sie Retinal in einer Konzentration von 0,04 bis 0,06 Gewichts-%, vorzugsweise etwa 0,05 Gewichts-%, und Propylgallat in einer Konzentration von 0,01 bis 0,03, vorzugsweise etwa 0,02 Gewichts-% enthält.

9. Zusammensetzung nach einem der Ansprüche 1 bis 8, **dadurch gekennzeichnet, dass** der pH der Zusammensetzung zwischen 6 und 7,5 eingeschlossen ist.

10. Verfahren zur Herstellung einer Zusammensetzung nach einem der Ansprüche 1 bis 9, **dadurch gekennzeichnet, dass** es die folgenden Schritte umfasst:
a) man bringt das Retinal in einem oder mehreren organischen und/oder Mineralölen, Wachsen, Estern und Siliconen in Lösung,
b) man gibt ein oder mehrere fettlösliche Antioxidantien zu der Lösung,
c) man emulgiert die erhaltene Lösung in einer wässrigen und/oder wässrig-alkoholischen Lösung, um eine Öl/Wasser-Emulsion zu erhalten.

## Claims

1. Dermocosmetological composition containing retinal, in 13-cis or 13-trans form or in the form of all other mixtures of these isomers, **characterized in that** it consists of an oil-in-water emulsion stabilized essentially by one or more fat-soluble antioxidants, the fat-soluble antioxidant/retinal concentration ratio being between 0.1 and 0.6.

2. Dermocosmetological composition according to Claim 1, **characterized in that** the fat-soluble antioxidant is chosen from the group comprising: butylated hydroxytoluene (BHT), butylated hydroxyanisole (BHA), ascorbyl palmitate, alpha-tocopherol and its esters, citric acid, propyl gallate and/or mixtures thereof.

3. Composition according to either of Claims 1 and 2, **characterized in that** the retinal is present in the oily phase.

4. Composition according to one of Claims 1 to 3, **characterized in that** the concentration of fat-soluble antioxidants in the oily phase is between 0.005% and 0.5% by weight.

5. Composition according to one of Claims 1 to 4, **characterized in that** the retinal concentration in the composition is between 0.01% and 1% by weight.

6. Composition according to one of Claims 1 to 5, **characterized in that** the fat-soluble antioxidant/retinal concentration ratio is approximately 0.4.

7. Composition according to one of Claims 1 to 6, **characterized in that** it contains retinal at a concentration of between 0.04% and 0.06% by weight, preferably of approximately 0.05%, and BHT at a concentration of between 0.01% and 0.03%, preferably of approximately 0.02%, by weight.

8. Composition according to one of Claims 1 to 6, **characterized in that** it contains retinal at a concentration of between 0.04% and 0.06% by weight, preferably of approximately 0.05%, and propyl gallate at a concentration of between 0.01% and 0.03%, preferably of approximately of 0.02%, by weight.

9. Composition according to one of Claims 1 to 8, **characterized in that** the pH of the composition is between 6 and 7.5.

10. Process for preparing a composition according to one of Claims 1 to 9, **characterized in that** it comprises the following steps:
a) retinal is dissolved in one or more organic and/or mineral oils, waxes, esters and silicones,
b) one or more fat-soluble antioxidants is/are added to the solution,
c) the solution obtained is emulsified in an aqueous and/or aqueous-alcoholic solution to obtain an oilin-water emulsion.
